# EUROPEAN PATENT APPLICATION

(11) **EP 4 813 284 A1**
(43) Date of publication of application: **30.09.2026**
(21) Application number: 25905660.4
(22) Date of filing: 14.05.2025
(51) Int. Cl.: A61B 1/307

(54) **RIGID-FLEXIBLE INTEGRATED ENDOSCOPE AND METHOD FOR USING SAME**

(30) Priority: 31.12.2024 CN 202411985828
(71) Applicant: Anqing Medical Co., Ltd, Shanghai 201201 (CN)
(72) Inventor: YAN, Hang, Shanghai 201201 (CN); TANG, Wei, Shanghai 201201 (CN)
(74) Representative: Argyma
(86) International application number: PCT/CN2025/094899
(87) International publication number: WO 2026/143947

(57) **Abstract**

A soft and hard integrated combination lens includes a hard sheath and an electronic soft endoscope. The electronic soft endoscope includes an insertion portion. The hard sheath includes a locking apparatus and a hard sheath catheter. The locking apparatus is disposed at a tail of the hard sheath catheter. A head end of the insertion portion is inserted into the hard sheath catheter from a tail end of the hard sheath catheter, and protrudes from a head end of the hard sheath catheter. The hard sheath catheter locks the insertion portion by using the locking apparatus. According to the present invention, the hard sheath and the electronic soft endoscope are combined to form a soft and hard integrated combination lens.

## Description

### TECHNICAL FIELD

The present invention relates to the field of medical device technologies, and in particular, to a soft and hard integrated combination lens and a use method therefor.

### BACKGROUND

Endoscopes have evolved from original optical and electronic mirrors to current safer or more convenient disposable electronic endoscopes. A disposable mirror replaces a multiplexing mirror, and a disposable ureteroscope in the urology field has the highest replacement rate. The disposable ureteroscope is mainly applicable to a ureteroscopic lithotripsy. With the ureteroscopic lithotripsy, an operation can be completed through a natural passage of a human body without puncturing. The ureteroscopic lithotripsy has advantages of a small trauma, a good lithotripsy effect, high stone clearing efficiency, and the like, and therefore is increasingly favored in clinical practice.

A rigid ureteroscope is easy to operate, but a front end of the rigid ureteroscope cannot be bent. As a result, the rigid ureteroscope cannot reach a renal pelvis through a ureter. Although a front end of a flexible ureteroscope can be bent, the flexible ureteroscope cannot be pushed directly from the ureter to the renal pelvis. To enable the flexible ureteroscope to reach the renal pelvis, the rigid ureteroscope is first placed into the human body through the ureter, then a guide wire is placed along a working passage of the rigid ureteroscope, then a soft lens sheath is placed along the guide wire, and finally a disposable flexible ureteroscope is inserted through an inner cavity of the soft lens sheath. The disposable flexible ureteroscope can reach the renal pelvis at most.

Because the rigid ureteroscope and the flexible ureteroscope usually do not share one image processor, a plurality of endoscope systems need to be provided for simultaneous use, increasing complexity and costs of an operational method. Even if the rigid ureteroscope and the flexible ureteroscope can share one image processor, an endoscope has to be switched back and forth, making the use inconvenient.

Therefore, how to develop a soft and hard integrated combination lens that integrates a rigid ureteroscope and a flexible ureteroscope becomes a technical issue that needs to be urgently addressed by persons skilled in the art.

### SUMMARY

To address the foregoing technical issue, an embodiment of the present invention provides a soft and hard integrated combination lens. The soft and hard integrated combination lens includes a hard sheath and an electronic soft endoscope. The electronic soft endoscope includes an insertion portion. The hard sheath includes a locking apparatus and a hard sheath catheter. The locking apparatus is disposed at a tail of the hard sheath catheter. A head end of the insertion portion is inserted into the hard sheath catheter from a tail end of the hard sheath catheter, and protrudes from a head end of the hard sheath catheter. The hard sheath catheter locks the insertion portion by using the locking apparatus.

Optionally, the head end of the hard sheath catheter is provided with a closure that is used to closely cooperate with the insertion portion.

Optionally, an outer diameter of the hard sheath catheter is less than 9.5 Fr and an inner diameter of the hard sheath catheter is greater than 8 Fr.

Optionally, the insertion portion includes a hose, and a head of the hose is a rigid head that is easy to be inserted into a human body.

Optionally, the rigid head is obliquely circular or bullet-shaped.

Optionally, an outer diameter of the hose is less than 8 Fr, and a diameter of a working passage of the hose is greater than 3.6 Fr.

Optionally, the locking apparatus includes a fixed portion and a movable portion, the fixed portion is fastened to the tail of the hard sheath catheter, and the movable portion and the fixed portion are cooperatively connected to lock the insertion portion.

Optionally, the fixed portion is a hollow sleeve structure, a head of the fixed portion is fixedly sleeved on the tail of the hard sheath catheter, and an external thread is disposed on an outer side of a tail of the fixed sleeve; the movable portion is a threaded sleeve, an internal thread fitting the external thread is disposed on an inner side of the threaded sleeve, and the insertion portion successively runs through the threaded sleeve and the fixed portion, and is tightened on the fixed portion by using the threaded sleeve, so as to clamp the insertion portion.

Optionally, the locking apparatus further includes an elastic locking member, the elastic locking member is sleeved on the insertion portion, and the movable portion and the fixed portion are cooperatively connected to compress the elastic locking member, so that the elastic locking member clamps the insertion portion.

Optionally, the fixed portion is a locking valve body structure, the locking valve body structure is a sleeve structure, an accommodating groove is disposed on an inner side of a tail of the sleeve structure, an inner diameter of the accommodating groove is greater than an inner diameter of a head of the sleeve structure, and the elastic locking member is located in the accommodating groove; the head of the sleeve structure is fixedly sleeved on the tail of the hard sheath catheter, an external thread is disposed on an outer side of the tail of the sleeve structure, an annular protrusion is further disposed on the outer side of the tail of the sleeve structure, and the annular protrusion is located at a head end of the external thread;
the movable portion is a locking valve cap structure, and is provided with a through hole for insertion of the insertion portion; a head of the locking valve cap structure includes an outer wall and an inner wall, and the inner wall forms the through hole; the outer wall is sleeved on an outer side of the inner wall at intervals, an internal thread fitting the external thread is disposed on an inner side of the outer wall, an annular clamping groove fitting the annular protrusion is further disposed on the inner side of the outer wall, and the annular clamping groove is located at a head end of the internal thread; and
during assembly, the tail of the sleeve structure extends between the outer wall and the inner wall of the locking valve cap structure, the annular protrusion is clamped into the annular clamping groove, the inner wall extends into the accommodating groove, the annular protrusion moves axially inside the annular clamping groove through tightening of the internal thread and the external thread, and a head end of the inner wall compresses the elastic locking member.

Another embodiment of the present invention further provides a method for using a soft and hard integrated combination lens. The method includes the following steps:
S1: inserting an insertion portion of the electronic soft endoscope into the hard sheath, so that a head end of the insertion portion protrudes from a head end of the hard sheath by a specific length, and locking the insertion portion onto the hard sheath by using the locking apparatus, to form the soft and hard integrated combination lens;
S2: placing the soft and hard integrated combination lens into a human body through a ureter;
S3: inserting a guide wire along a working passage of the electronic soft endoscope;
S4: withdrawing the soft and hard integrated combination lens; and
S5: removing the hard sheath from the soft and hard integrated combination lens, and inserting the electronic soft endoscope along the guide wire into a renal pelvis.

Compared with a conventional technology, the technical solutions in the embodiments of the present invention have the following beneficial effects:
According to the present invention, the hard sheath and the electronic soft endoscope are combined to form a soft and hard integrated combination lens. With the soft and hard integrated combination lens, use of an endoscope may be reduced, and use of an endoscope system may be further reduced, thereby reducing complexity and costs of an operational method, and making the use convenient, safe, and quick.

### BRIEF DESCRIPTION OF DRAWINGS

To describe the technical solutions in the embodiments of the present invention or in a conventional technology more clearly, the following briefly describes the accompanying drawings required for describing the embodiments or the conventional technology. Clearly, the accompanying drawings in the following description show merely some embodiments of the present invention, and persons of ordinary skill in the art may still derive other drawings from these accompanying drawings without creative efforts.
FIG. 1 is a schematic structural diagram of a soft and hard integrated combination lens according to an embodiment of the present invention;
FIG. 2 is a schematic structural diagram of an electronic soft endoscope according to an embodiment of the present invention;
FIG. 3 is a schematic structural diagram of a hard sheath according to an embodiment of the present invention;
FIG. 4 is an exploded view of a hard sheath according to an embodiment of the present invention;
FIG. 5 is a schematic structural diagram of a locking apparatus that does not lock an insertion portion according to an embodiment of the present invention; and
FIG. 6 is a schematic structural diagram of a locking apparatus that locks an insertion portion according to an embodiment of the present invention.

### DESCRIPTION OF EMBODIMENTS

The following clearly and comprehensively describes the technical solutions in the embodiments of the present invention with reference to the accompanying drawings in the embodiments of the present invention. Clearly, the described embodiments are merely some rather than all of the embodiments of the present invention. All other embodiments obtained by persons of ordinary skill in the art based on the embodiments of the present invention without creative efforts shall fall within the protection scope of the present invention.

In the specification, claims, and accompanying drawings of the present invention, the terms "first", "second", "third", "fourth", and the like (if existent) are intended to distinguish between similar objects, and are not necessarily intended to describe a specific order or sequence. It should be understood that the data used in such a way is interchangeable in proper circumstances so that the embodiments of the present invention described herein can be implemented in other orders than the order illustrated or described herein. In addition, the terms "include", "comprise", "have", and any other variants thereof are intended to cover non-exclusive inclusion. For example, a process, method, system, product, or device that includes a series of steps or units is unnecessarily limited to those expressly listed steps or units, but may include other steps or units that are not expressly listed or that are inherent to such a process, method, system, product, or device. The terms "up", "upper", and any other variants thereof are intended to describe the positional relationship and do not represent that there is a direct contact relationship between described objects.

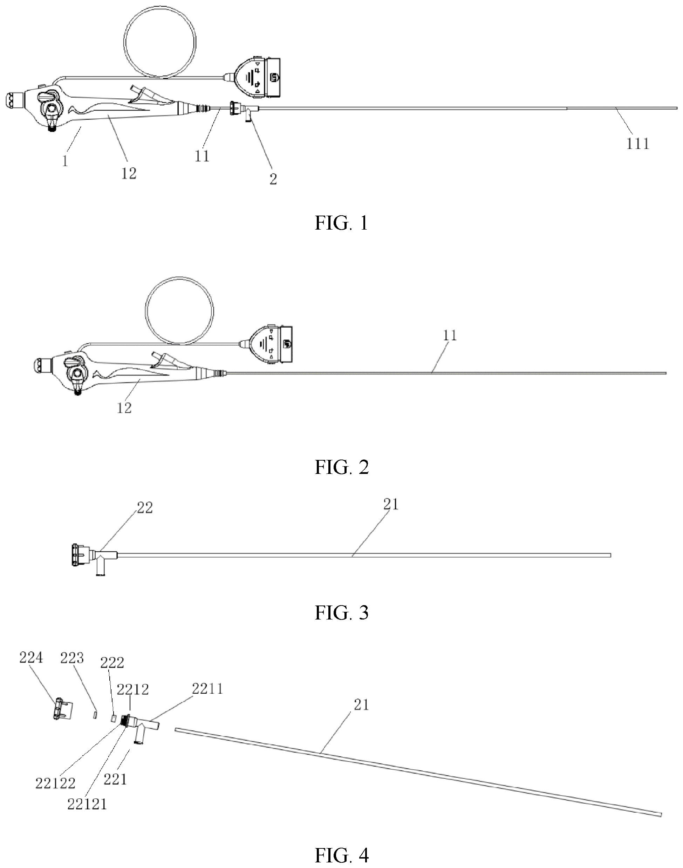

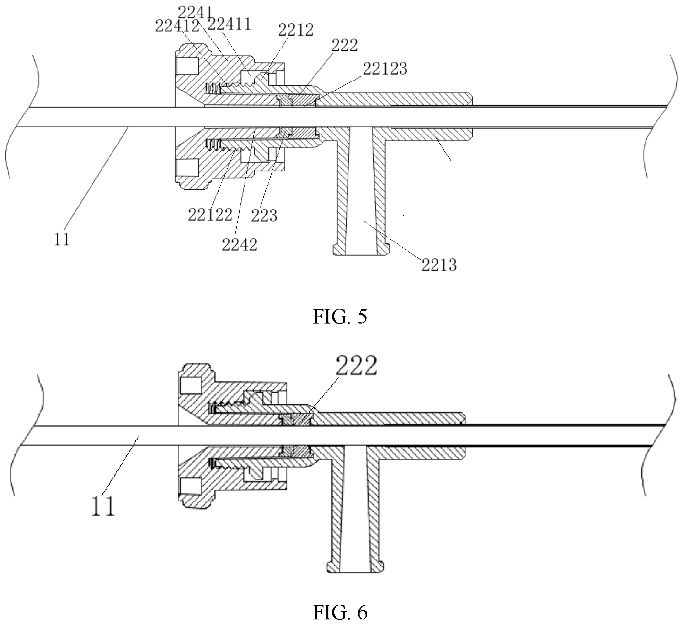

Referring to FIG. 1 to FIG. 6, an embodiment of the present invention provides a soft and hard integrated combination lens. The soft and hard integrated combination lens includes a hard sheath 2 and an electronic soft endoscope 1.

To ensure safety and hygiene, and avoid cross-infection, in this embodiment, both the hard sheath 2 and the electronic soft endoscope 1 are preferably disposable.

The electronic soft endoscope 1 belongs to a mature technology in the art. Therefore, the present invention does not provide much description of the electronic soft endoscope. The electronic soft endoscope 1 includes an operation portion 12 and an insertion portion 11 used for insertion into a human body, and the operation portion 12 is located at a tail of the insertion portion 11.

The hard sheath 2 includes a locking apparatus 22 and a hard sheath catheter 21. The hard sheath catheter 21 is a hollow tubular structure. To ensure hard rigidity, the hard sheath catheter 21 is made of a hard material, such as a metal material. The locking apparatus 22 is disposed at a tail of the hard sheath catheter 21.

In this embodiment, the insertion portion 11 of the electronic soft endoscope 1 is detachably fastened to the hard sheath catheter 21 by using the locking apparatus 22. Specifically, a head end of the insertion portion 11 is inserted into the hard sheath catheter 21 from a tail end of the hard sheath catheter 21, and protrudes from a head end of the hard sheath catheter 21. The hard sheath catheter 21 locks the insertion portion 11 by using the locking apparatus 22, to form a soft and hard integrated combination lens.

When the soft and hard integrated combination lens is inserted into the human body, to prevent scratching of the human body caused by a gap between the head end of the hard sheath catheter 21 and the insertion portion 11, the head end of the hard sheath catheter 21 is provided with a closure that is used to closely cooperate with the insertion portion 11, that is, the head end of the hard sheath catheter 21 is tightly attached to the insertion portion 11 by using the closure, so that there is no gap between the head end of the hard sheath catheter 21 and the insertion portion 11.

Formation of the closure is not limited in the present invention. For example, the port may be bullet-shaped or obliquely circular.

A smaller outer diameter of the hard sheath catheter 21 leads to easier insertion into a passage of the human body. Therefore, the outer diameter of the hard sheath catheter 21 is preferably less than 9.5 Fr. Similarly, an outer diameter of the insertion portion 11 of the electronic soft endoscope 1 is less than 8 Fr. To enable the insertion portion 11 of the electronic soft endoscope 1 to be inserted into the hard sheath catheter 21, an inner diameter of the insertion portion 11 of the electronic soft endoscope 1 is greater than 8 Fr.

A working passage is further disposed inside the insertion portion 11 of the electronic soft endoscope 1, and a diameter of the working passage is greater than 3.6 Fr, so as to facilitate an operation.

In this embodiment, the insertion portion 11 of the electronic soft endoscope 1 includes a hose. Because the hose is not easy to be inserted into a passage of the human body, a head of the hose is designed as a rigid head 111 that is easy to be inserted into the human body, and the rigid head 111 is made of a hard material, such as metal. When the electronic soft endoscope 1 is assembled with the hard sheath 2, the rigid head 111 of the hose protrudes from the head end of the hard sheath catheter 21 by a specific length, and the length protruding from the head end of the hard sheath catheter 21 is less than a length of the rigid head 111, so as to prevent the hose from protruding from the head end of the hard sheath catheter 21. The length of the rigid head 111 is not specifically limited in this embodiment. Generally, the length of the rigid head 111 protruding from the head end of the hard sheath catheter 21 needs to be 3-6 mm.

A shape of the rigid head 111 is not limited in the present invention, provided that the rigid head is rounded and easy to be inserted into the human body. For example, the rigid head 111 is obliquely circular, bullet-shaped, or cylindrical.

A specific structure of the locking apparatus 22 is not limited in the present invention, provided that the electronic soft endoscope 1 can be locked on the hard sheath 2 and the electronic soft endoscope 1 can be unlocked from the hard sheath 2.

In an embodiment, the locking apparatus 22 includes a fixed portion 221 and a movable portion 224, the fixed portion 221 is fastened to the tail of the hard sheath catheter 21, and the movable portion 224 and the fixed portion 221 are cooperatively connected to lock the insertion portion 11. The cooperative connection between the movable portion 224 and the fixed portion 221 may be a detachable connection manner such as a threaded fastening connection or/and a clamping connection.

In a specific implementation, the fixed portion 221 is a hollow sleeve structure, a head of the fixed portion 221 is fixedly sleeved on the tail of the hard sheath catheter 21, and an external thread is disposed on an outer side of a tail of the fixed sleeve; the movable portion 224 is a threaded sleeve, an internal thread fitting the external thread is disposed on an inner side of the threaded sleeve, and the insertion portion 11 successively runs through the threaded sleeve and the fixed portion 221, and is tightened on the fixed portion 221 by using the threaded sleeve, so as to clamp the insertion portion 11.

In this embodiment, the tail of the fixed sleeve may be an elastic body, or may be provided with several axially disposed notches on an outer periphery. A purpose is as follows: When the threaded sleeve is tightened on the fixed portion 221, the threaded sleeve squeezes the tail of the fixed portion 221, so that a diameter of the fixed portion becomes smaller, and further the fixed portion can clamp the insertion portion 11 to lock the insertion portion 11.

In another specific implementation, the locking apparatus 22 further includes an elastic locking member 222, the elastic locking member 222 is sleeved on the insertion portion 11, and the movable portion 224 and the fixed portion 221 are cooperatively connected to compress the elastic locking member 222, so that the elastic locking member 222 clamps the insertion portion 11.

The elastic locking member 222 is not limited in the present invention, provided that the elastic locking member can be compressed by the movable portion 224 and the fixed portion 221 to generate deformation, so as to clamp the insertion portion 11.

In this embodiment, the elastic locking member 222 is a soft rubber ring, and in an uncompressed state, there is a gap between the elastic locking member 222 and the insertion portion 11. Therefore, the insertion portion 11 may move axially relative to the hard sheath 2. When being pressed by the movable portion 224 and the fixed portion 221, the elastic locking member 222 generates deformation to clamp the insertion portion 11, so that the insertion portion 11 cannot move axially relative to the hard sheath 2.

The cooperative connection between the movable portion 224 and the fixed portion 221 may be a threaded connection, a clamping connection, or/and the like.

Specifically, the fixed portion 221 is a locking valve body structure, the locking valve body structure is a sleeve structure, an accommodating groove is disposed on an inner side of a tail 2212 of the sleeve structure, an inner diameter of the accommodating groove is greater than an inner diameter of a head 2211 of the sleeve structure, and the elastic locking member 222 is located in the accommodating groove; the head 2211 of the sleeve structure is fixedly sleeved on the tail of the hard sheath catheter 21, an external thread 22122 is disposed on an outer side of the tail 2212 of the sleeve structure, an annular protrusion 22121 is further disposed on the outer side of the tail 2212 of the sleeve structure, and the annular protrusion 22121 is located at a head end of the external thread 22122.

The movable portion 224 is a locking valve cap structure, and is provided with a through hole for insertion of the insertion portion 11; a head of the locking valve cap structure includes an outer wall 2241 and an inner wall 2242, and the inner wall 2242 forms the through hole; the outer wall 2241 is sleeved on an outer side of the inner wall 2242 at intervals, an internal thread 22412 fitting the external thread 22122 is disposed on an inner side of the outer wall 2241, an annular clamping groove 22411 fitting the annular protrusion 22121 is further disposed on the inner side of the outer wall 2241, and the annular clamping groove 22411 is located at a head end of the internal thread 22412. An axial length of the annular clamping groove 22411 is greater than an axial length of the annular protrusion 22121, so that the annular protrusion 22121 can move axially inside the annular clamping groove 22411 when the elastic locking member 222 does not lock the insertion portion 11.

During assembly, first, the tail 2212 of the sleeve structure extends between the outer wall 2241 and the inner wall 2242 of the locking valve cap structure, the annular protrusion 22121 is clamped into the annular clamping groove 22411, and the inner wall 2242 extends into the accommodating groove. In this case, the elastic locking member 222 does not lock the insertion portion 11, there is still a gap 22123 between the insertion portion 11 and the inner wall 2242, and the insertion portion 11 can still move axially relative to the hard sheath 2. Then, the annular protrusion 22121 moves axially inside the annular clamping groove 22411 through tightening of the internal thread 22412 and the external thread 22122, and a head end of the inner wall 2242 compresses the elastic locking member 222, so that the elastic locking member 222 clamps the insertion portion 11, and the insertion portion 11 can no longer move axially relative to the hard sheath 2.

To facilitate compression of the elastic locking member 222, a spacer ring 223 is further disposed between the head end of the inner wall 2242 and the elastic locking member 222, and the head end of the inner wall 2242 compresses the elastic locking member 222 by using the spacer ring 223, so that the elastic locking member 222 clamps the insertion portion 11.

In an embodiment of the present invention, a lateral passage 2213 is further disposed on a side surface of the fixed portion 221 of the sleeve structure.

Another embodiment of the present invention further provides a method for using a soft and hard integrated combination lens. The method includes the following steps:
S1: inserting an insertion portion 11 of the electronic soft endoscope 1 into the hard sheath 2, so that a head end of the insertion portion 11 protrudes from a head end of the hard sheath 2 by a specific length, and locking the insertion portion 11 onto the hard sheath 2 by using the locking apparatus 22, to form the soft and hard integrated combination lens;
S2: placing the soft and hard integrated combination lens into a human body through a ureter;
S3: inserting a guide wire along a working passage of the electronic soft endoscope 1;
S4: withdrawing the soft and hard integrated combination lens; and
S5: removing the hard sheath 2 from the soft and hard integrated combination lens by using the locking apparatus 22, and inserting the electronic soft endoscope 1 along the guide wire into a renal pelvis.

According to the present invention, the hard sheath 2 and the electronic soft endoscope 1 are combined to form a soft and hard integrated combination lens. With the soft and hard integrated combination lens, use of an endoscope may be reduced, and use of an endoscope system may be further reduced, thereby reducing complexity and costs of an operational method, and making the use convenient, safe, and quick.

Finally, it should be noted that the foregoing embodiments are merely intended for describing the technical solutions of the present invention, but not for limiting the present invention. Although the present invention is described in detail with reference to the foregoing embodiments, persons of ordinary skill in the art should understand that they may still make modifications to the technical solutions described in the foregoing embodiments or make equivalent replacements to some or all technical features thereof, without departing from the scope of the technical solutions in the embodiments of the present invention.

## Claims

1. A soft and hard integrated combination lens, comprising a hard sheath and an electronic soft endoscope, wherein the electronic soft endoscope comprises an insertion portion; the hard sheath comprises a locking apparatus and a hard sheath catheter; the locking apparatus is disposed at a tail of the hard sheath catheter; a head end of the insertion portion is inserted into the hard sheath catheter from a tail end of the hard sheath catheter, and protrudes from a head end of the hard sheath catheter; and the hard sheath catheter locks the insertion portion by using the locking apparatus.

2. The soft and hard integrated combination lens according to claim 1, wherein the head end of the hard sheath catheter is provided with a closure that is used to closely cooperate with the insertion portion.

3. The soft and hard integrated combination lens according to claim 1, wherein an outer diameter of the hard sheath catheter is less than 9.5 Fr and an inner diameter of the hard sheath catheter is greater than 8 Fr.

4. The soft and hard integrated combination lens according to claim 1, wherein the insertion portion comprises a hose, and a head of the hose is a rigid head that is easy to be inserted into a human body.

5. The soft and hard integrated combination lens according to claim 4, wherein the rigid head is obliquely circular or bullet-shaped.

6. The soft and hard integrated combination lens according to claim 4, wherein an outer diameter of the hose is less than 8 Fr, and a diameter of a working passage of the hose is greater than 3.6 Fr.

7. The soft and hard integrated combination lens according to claim 1, wherein the locking apparatus comprises a fixed portion and a movable portion, the fixed portion is fastened to the tail of the hard sheath catheter, and the movable portion and the fixed portion are cooperatively connected to lock the insertion portion.

8. The soft and hard integrated combination lens according to claim 7, wherein the fixed portion is a hollow sleeve structure, a head of the fixed portion is fixedly sleeved on the tail of the hard sheath catheter, and an external thread is disposed on an outer side of a tail of the fixed sleeve; the movable portion is a threaded sleeve, an internal thread fitting the external thread is disposed on an inner side of the threaded sleeve, and the insertion portion successively runs through the threaded sleeve and the fixed portion, and is tightened on the fixed portion by using the threaded sleeve, so as to clamp the insertion portion.

9. The soft and hard integrated combination lens according to claim 7, wherein the locking apparatus further comprises an elastic locking member, the elastic locking member is sleeved on the insertion portion, and the movable portion and the fixed portion are cooperatively connected to compress the elastic locking member, so that the elastic locking member clamps the insertion portion.

10. The soft and hard integrated combination lens according to claim 9, wherein the fixed portion is a locking valve body structure, the locking valve body structure is a sleeve structure, an accommodating groove is disposed on an inner side of a tail of the sleeve structure, an inner diameter of the accommodating groove is greater than an inner diameter of a head of the sleeve structure, and the elastic locking member is located in the accommodating groove; the head of the sleeve structure is fixedly sleeved on the tail of the hard sheath catheter, an external thread is disposed on an outer side of the tail of the sleeve structure, an annular protrusion is further disposed on the outer side of the tail of the sleeve structure, and the annular protrusion is located at a head end of the external thread;
the movable portion is a locking valve cap structure, and is provided with a through hole for insertion of the insertion portion; a head of the locking valve cap structure comprises an outer wall and an inner wall, and the inner wall forms the through hole; the outer wall is sleeved on an outer side of the inner wall at intervals, an internal thread fitting the external thread is disposed on an inner side of the outer wall, an annular clamping groove fitting the annular protrusion is further disposed on the inner side of the outer wall, and the annular clamping groove is located at a head end of the internal thread; and
during assembly, the tail of the sleeve structure extends between the outer wall and the inner wall of the locking valve cap structure, the annular protrusion is clamped into the annular clamping groove, the inner wall extends into the accommodating groove, the annular protrusion moves axially inside the annular clamping groove through tightening of the internal thread and the external thread, and a head end of the inner wall compresses the elastic locking member.

11. A method for using the soft and hard integrated combination lens according to any one of claims 1 to 10, comprising the following steps:
S1: inserting an insertion portion of the electronic soft endoscope into the hard sheath, so that a head end of the insertion portion protrudes from a head end of the hard sheath by a specific length, and locking the insertion portion onto the hard sheath by using the locking apparatus, to form the soft and hard integrated combination lens;
S2: placing the soft and hard integrated combination lens into a human body through a ureter;
S3: inserting a guide wire along a working passage of the electronic soft endoscope;
S4: withdrawing the soft and hard integrated combination lens; and
S5: removing the hard sheath from the soft and hard integrated combination lens, and inserting the electronic soft endoscope along the guide wire into a renal pelvis.
